# EUROPEAN PATENT SPECIFICATION

(11) **EP 0 491 793 B1**
(45) Date of publication and mention of the grant of the patent: **30.11.2005**
(21) Application number: 90913760.6
(22) Date of filing: 14.09.1990
(51) Int. Cl.: C07H 19/04, A61K 31/70

(54) **2'-DEOXY-4'-THIORIBONUCLEOSIDES AS ANTIVIRAL AND ANTICANCER AGENTS**
2'-DEOXY-4'-THIORIBONUCLEOSIDE ALS ANTIVIRALE UND ANTIKREBSMITTEL
2'-DESOXY-4'-THIORIBONUCLEOSIDES UTILES COMME AGENTS ANTIVIRAUX ET ANTICANCEREUX

(30) Priority: 15.09.1989 US 408040
(43) Date of publication of application: 01.07.1992
(73) Proprietor: Southern Research Institute, Birmingham Alabama 35255-5305 (US)
(72) Inventor: MONTGOMERY, John, A., Birmingham, AL 35233 (US); SECRIST, John, A., III, Birmingham, AL 35242 (US)
(74) Representative: Leissler-Gerstl, Gabriele
(86) International application number: PCT/US1990/005252
(87) International publication number: WO 1991/004033

(56) References cited:
- EP-A- 0 316 017
- WO-A-88/08001
- JOURNAL OF MEDICINAL CHEMISTRY vol. 19, no. 6, 1976, WASHINGTON US pages 841 - 2 M.V.PICKERING ET AL. 'Synthesis of 1-(4-Th io-B-D-ribofuranosyl)-1,2,4-triazole-3-car boxamide.'
- JOURNAL OF THE CHEMICAL SOCIETY, CHEMICAL COMMUNICATIONS vol. 18, 1973, LETCHWORTH GB pages 686 - 7 G.S.RITCHIE ET AL. 'Synthesis of 4'-Thiocordycepin.'
- J. Am. Chem. Soc. 86, Issued 20 December 1984, (Am. Chem. Soc.) Easton, PA, REIST et al., "Synthesis of 4-Thio-D-and L-ribofuranose and the Corresponding Adenine Nucleosides", see whole document, pages 5658-5663.
- Purine and Pyrimidine Metabolism in Man, Volume V,. Part B, NYHAN et al. eds., Issued 1986, (Plenum Publishing Corporation) New York, MIURA et al., "4'-thioadenosine as a Novel Inhibitor of S-Adenosylhomocysteine Hydrolase and an Inducer for the Differentiation of HL-60 Human Leukemia Cells", see whole document.
- J. Organic Chemistry, Vol. 33(1), Issued January 1968, (Am. Chem. Soc.) Easton, PA, REIST et al., "Thio Sugars, Synthesis of Adenine Nucleosides of 4-Thio-D-xylose and 4-Thio-D-arabinose", see whole document., page 189-190.
- J. Medicinal Chem., Vol. 17(5), Issued 1974, (Am. Chem. Soc.) Easton, PA, OTOTANI et al., "Preparation and Antitumor Activity of 4'-Thio Analogs of 2,2'-Anhydro-1-beta-D-arabinofuranosylcytosine", see whole document., page 535-537.
- Canadian J. Chem., Volume 56, Issued 1977, Canada, RICHIE et al., "Addition of Pseudohalogens to Unsaturated Carbohydrates, VI. Synthesis of 4'-thiocordycepin", see whole document, pp. 794-802.
- J., Organic Chem., Vol. 41(24), Issued 1976, (Am. Chem. Soc.) Easton, PA, FU et al., "An Alternative Synthesis of Anomeric Methyl 2-Deoxy-4-thio-D-erythro-pentofuranosides", see whole document, pages 3831-3834.
- J. Organic Chem., Vol. 35(2), Issued February 1970, (Am. Chem. Soc.) Easton, PA, WHISTLER et al., "Anomeric Methyl 4-thio-D-arabinofuranosides", see whole document., pages 519-521.
- FULMER SHEALY Y. ET AL: 'Carbocyclic analogs of 5-fluorouracil nucleosides' J.MED.CHEM. vol. 24, no. 9, 1981, pages 1083 - 1086
- J.CARBOHYDRATES. NUCLEOSIDES NUCLEOTIDES vol. 5, no. 3, 1978, pages 187 - 224

## Description

### Background of the Invention

### 1. Field of the invention

This invention relates to β-2'-deoxy-4'-thioribonucleosides and intermediates useful in their production, and to the use of β-2'-de oxy-4'-thioribonucleosides as antiviral and anticancer agents.

A nucleoside is a molecule comprised of a pentose sugar in a furanose ring joined to a nitrogenous heterocyclic base that is a derivative of either purine or pyrimidine. A 4'-thionucleoside is a nucleoside wherein the furanose ring oxygen has been replaced by sulfur. A 2'-deoxy-4'-thioribonucleoside is a 4'-thionucleoside wherein the pentose sugar is 2'-deoxy-D-ribose.

As used herein, the terms "nucleoside", "4'-thionucleoside" and "2'-deoxy-4'-thioribonucleoside" shall also include compounds wherein the nitrogenous heterocyclic base is a base related to the pyrimidine base, but with a ring alteration, such nitrogenous heterocyclic bases including 5-azapyrimidines, 6-azapyrimidines, and 3-deazapyrimidines and shall also include compounds having acyl protecting groups at the 3' position, or the 5' position, or both, of the 2'-deoxy-D-ribose.

### 2. Description of the Related Art

Several 4'-thionucleosides have been reported in the literature. Reist, et al, J. Am. Chem. Soc., 86, 5658 (1964) disclose L and D forms of 4'-thioriboadenosine. Biological effects of 4'-thioriboadenosine are described in Miura, et al in Purine and Pyrimidine Metabolism in Man, V, Part B, (Plenum Publishing Corp., 1986) p. 667. Richie, et al, Can. J. Chem., 56, 794 (1977) disclose the synthesis of 9-(3-deoxy-4-thio-β-D-erythro-pentofuranosyl)adenine (4'-thiocordycepin). Reist, et al, J. Org. Chem., 33, 189 (1968) describe the synthesis of adenine nucleosides of 4-thio-D-xylose and 4-thio-D-arabinose. Ototani, et al. J. Med. Chem., 17, 535 (1974) disclose the preparation and antitumor activity of 4' -thio-1-β-D-arabinofuranosylcytosine and 2,2'-anhydro-4'-thio-1-β-D-arabinofuranosylcytosine hydrochloride.

The description, preparation and use of specific 2'-deoxy-4'-thioribonucleosides is not found in the literature. Fu, et al, J. Org. Chem., 41, 3831 (1976) disclose a method for the preparation of anomeric methyl-2-deoxy-4-thio-D-erythro-pentofuranosides and suggest that the furanosides could be used as precursors for the synthesis of 2'-deoxy-4'-thionucleosides.

The non-prepublished European patent application EP 0 409 575 discloses anit-viral pyrimidine nucleosides wherein the sugar moiety is the 2-deoxy-4-thio-D-ribofuranose moiety. The general formulae disclosed in EP 0 409 575 comprises a multiplicity of compounds, inter alia a 5-methyl-uracil.

### Summary of the Invention

It has now been found that certain 2'-deoxy-4'-thioribonucleosides have useful antiviral and anticancer activities. Thus, in accordance with this invention, there are provided novel β-2'-deoxy-4'-thioribonucleosides represented by the formula 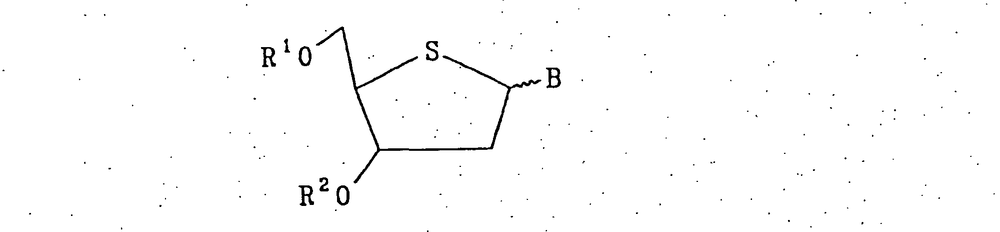 wherein:
~B indicates that B is beta, and
B is a nitrogenous heterocyclic base selected from the group consisting of pyrimidine, 5-azapyrimidine, 6-azapyrimidine, 3-deazapyrimidine bases. By the term "pyrimidine base" is meant any, pyrimidine derivative including, but not limited to, uracil (2,4-dioxopyrimidine), thymine (5-methyl-2,4-dioxopyrimidine), cytosine (4-amino-2-oxopyrimidine), and 5-methylcytosine (4-amino-5-methyl-2-oxopyrimidine),. By the term "5-azapyrimidine base" is meant any 5-azapyrimidine derivative including, but not limited to, 5-aza-2,4-dioxopyrimidine and 4-amino-5-aza-2-oxopyrimicine. By the term "6-azapyrimidine base" is meant any 6-azapyrimidine derivative including, but not limited to, 5-aza-2,4-dioxopyrimidine, 4-amino-6-aza-2-oxopyrimidine, and derivatives having a methyl group attached to the C⁵ heterocyclic carbon. By the term "3-deazapyrimidine base" is meant any 3-deazapyrimidine derivative including, but not limited to, 3-deaza-2,4-dioxopyrimidine, 4-amino-3-deaza-2-oxopyrimidine, and derivatives having a methyl group attached to the C⁵ heterocyclic carbon.
R¹ and R² in the above diagram may be the same or different and may be hydrogens or conventional acyl protecting groups.

The invention may be illustrated by the following, wherein the compounds encompassed by the invention are represented by the formula 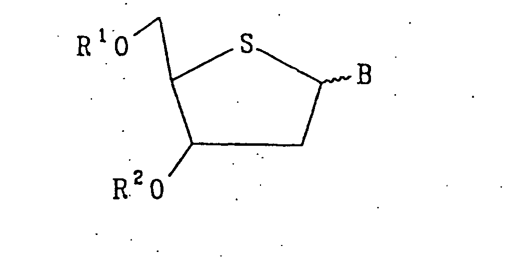 wherein:
~B indicates that B is beta, and
B is a member selected from the group consisting of the following nitrogenous heterocyclic bases: 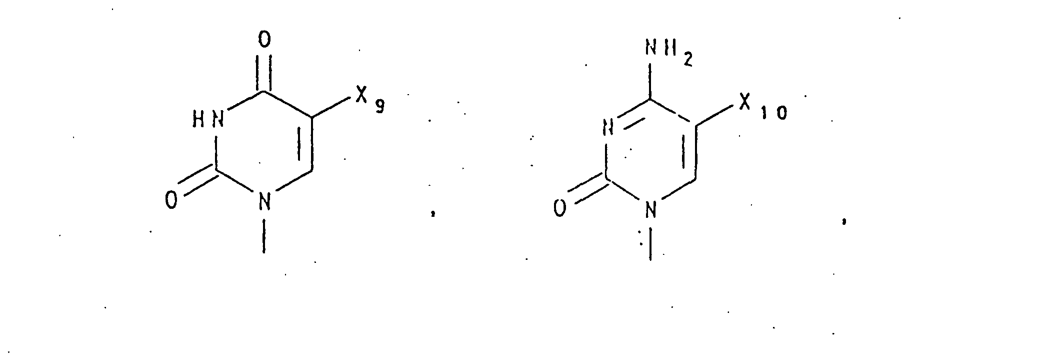 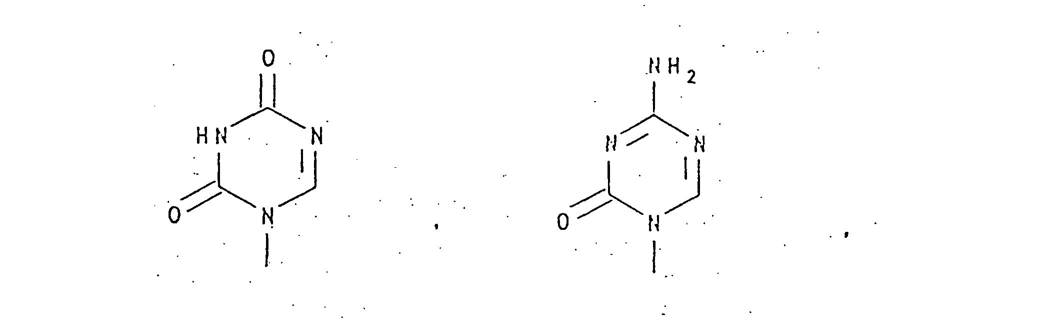 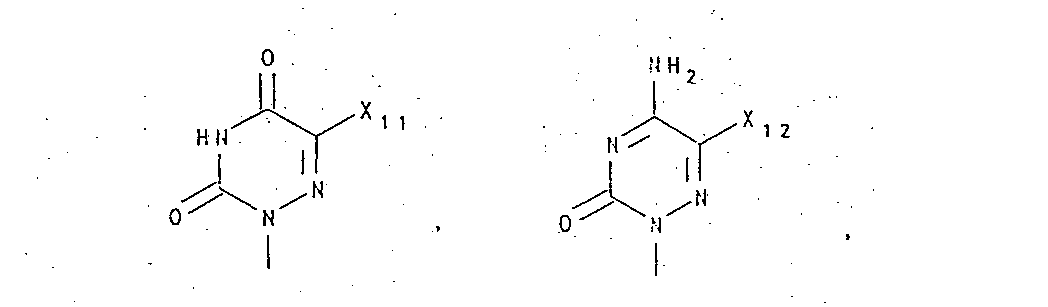 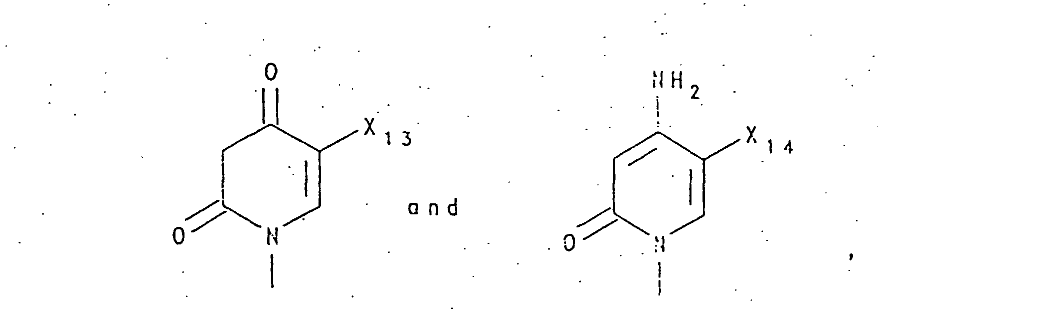
where X₉, X₁₀, X₁₁, X₁₂, X₁₃, X₁₄ =H, or CH₃, and R¹ and R² can be the same or different and may be hydrogen or acyl protecting groups, provided that it is not uracil having a methyl group attached to the C⁵ heterocyclic carbon.

Preferably, the nitrogenous heterocyclic base is selected from the group consisting of the following pyrimidine bases: 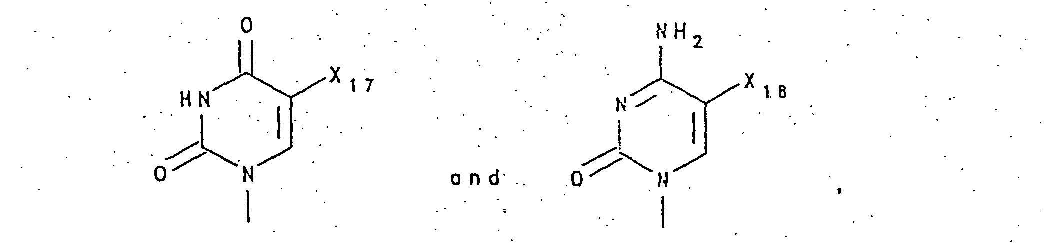 where X₁₇, X₁₈ =H or CH₃

According to another aspect of this invention, a therapeutically effective amount of a 2'-deoxy-4'-thioribonucleoside as previously defined is used to be administered to a host animal, including man, afflicted with a viral infection, e.g., an infection caused by herpes simplex virus types 1 or 2.

According to another aspect of this invention, a therapeutically effective amount of a 2'-deoxy-4'-thioribonucleoside as previously defined is used to be administered to a host animal, including man, afflicted with cancer. By the term "cancer" is meant any new and abnormal cell growth, specifically a new growth of tissue which is uncontrolled and progressive. Compounds of this invention may be used, for example, in the treatment of leukemias, epidermoid carcinoma, lymphomas, choriocarcinoma, Wilm's tumor, neuroblastoma, rhabdomyosarcoma, carcinoma of the testis, and breast and lung tumors.

In accordance with still another aspect of this invention, there are provided novel intermediates useful in the preparation of certain 2'-deoxy-4,'-thioribonucleosides.

### Detailed Description of the Invention

The synthesis of the 2'-deoxy-4'-thioribonucleosides may be carried out by beginning with 1-O-methyl 2-deoxy-4-thio-α,β-D-ribofuranose of formula 1 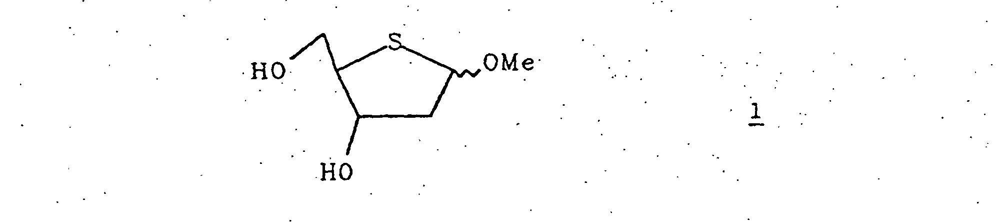 the preparation of which is described in Fu, et al, J. Org. Chem., 41, 3831 (1976), the disclosure of which is incorporated herein by reference. The compound of formula 1 is reacted with p-toluoyl chloride to give the compound of formula 2

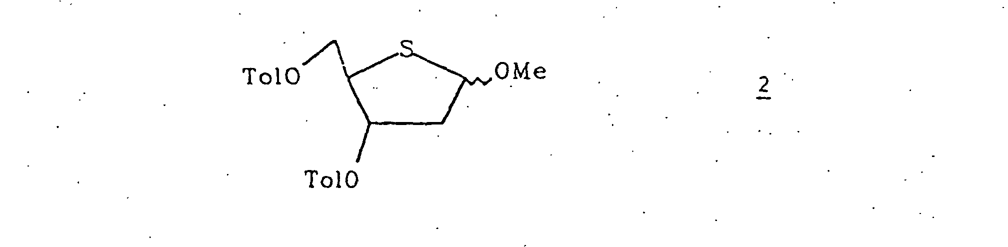

The methyl group is then replaced by an acetyl group to give the compound of formula 3

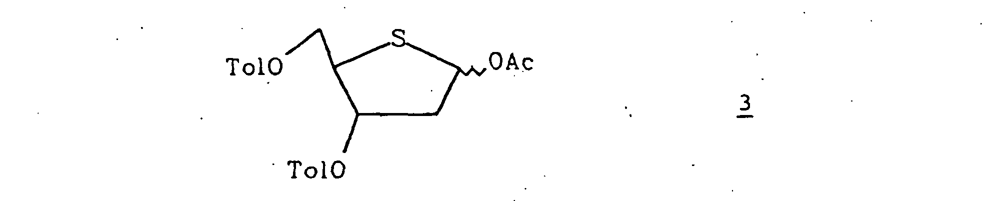

The 2'-deoxy-4'-thioribonucleosides are prepared by coupling compound 3 with nitrogenous heterocyclic bases and then removing the toluoyl protecting groups. Compound 3 is coupled with a pyrimidine using the catalysts hexamethyldisilazane, trimethylchlorosilane, and trimethylsilyl trifluoromethanesulfonate (see Vorbruggen, et al, Chem. Ber., 114, 1234 (1981)). Compound 3 is coupled with a 5-azapyrimidine, 6-azapyrimidine and 3-deazapyrimidine in a similar manner. The coupling reaction provides both α and β nucleosides in most cases. Anomers may be separated by conventional methods.

As examples of the preparation of pyrimidine compounds of this invention, the compound of formula 3 can be combined with uracil to give the compound of formula 15

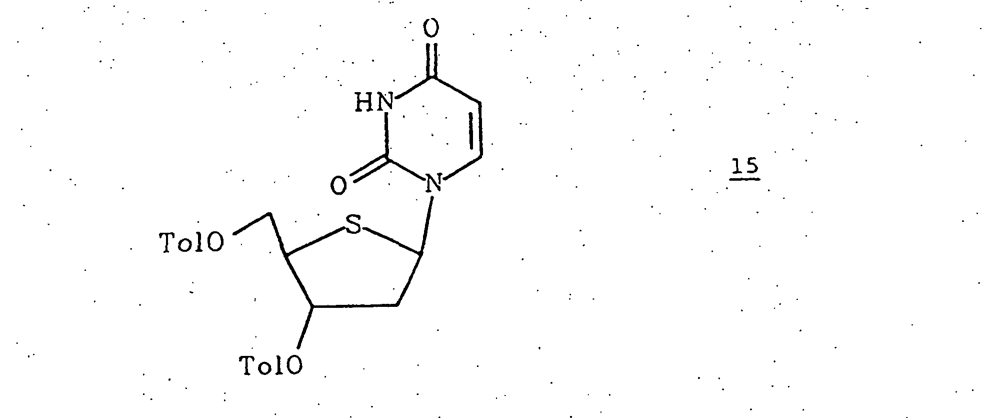

Removal of the toluoyl protecting groups gives the compound of the formula 16

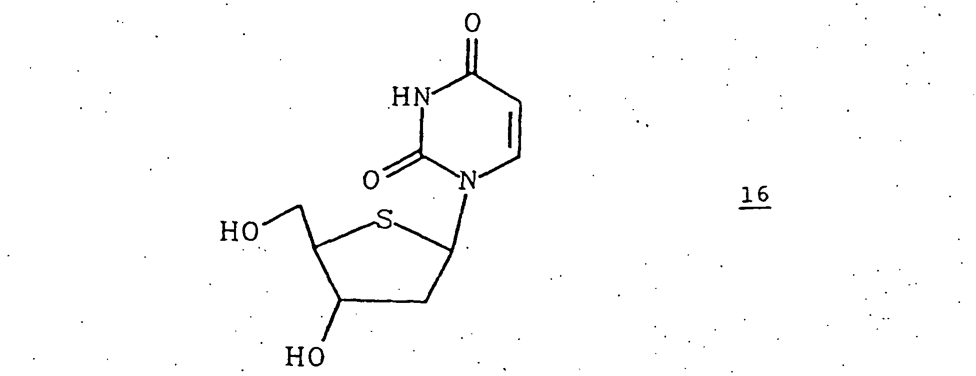

The compound of formula 3 can also be combined with thymine to give the compound of formula 17

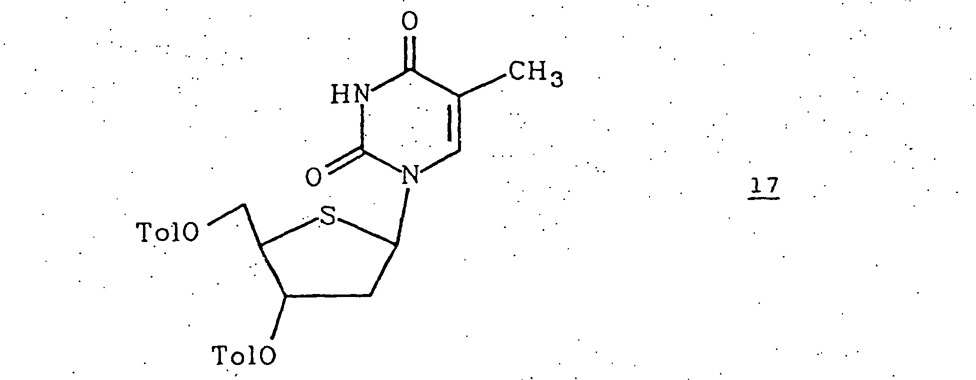

Removal of the toluoyl protecting groups gives the compound of formula 18

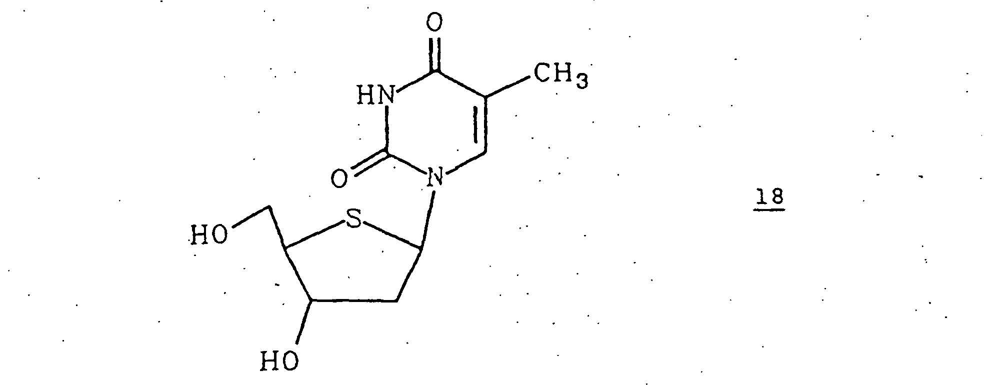

In carrying out the synthesis of 2'-deoxy-4'-thioribonucleosides of this invention, other acyl protecting groups besides the toluoyl protecting group may be used. Further, conventional acyl protecting groups may be substituted.or added, using conventional methods, to the 3' position or 5' position, or both, of the 2'-deoxy-4'-thioribonucleosides.

The following examples illustrate the preparation of the compounds described above. In these examples, MeOH is methyl alcohol, EtOH is ethyl alcohol, and Me₂SO-d₆ is deuterated dimethyl sulfoxide (CD₃)₂SO.

### EXAMPLE 1

1-O-Acetyl-2-deoxy-4-thio-3,5-di-O-p-toluoyl-α,β-D-ribofuranose (formula 3). To a solution of 1-O-methyl-2-deoxy-4-thio-α,β-D-ribofuranose (formula 1) (10 g, 60.97 mmol) in 250 mL of sieve-dried pyridine was added p-toluoyl chloride (23.57 g, 152.5 mmol) dropwise at 0-5°C. The cooling bath was removed. After the reaction stirred for 10 hours, the reaction was essentially complete as indicated by TLC (cyclohexane-ethylacetate 5:1). The reaction mixture was poured into an ice-water mixture, stirred for 1 hour, and then diluted with 500 mL of CHCl₃ to give a total volume of 1000 mL. The aqueous layer was extracted with CHCl₃ (2 x 100 mL). The combined organic extracts were washed with dilute sulfuric acid (200 mL), aqueous saturated sodium bicarbonate (2 x 200 mL) and water until neutral, dried over MgSO₄, and evaporated to dryness. The residue was dissolved in CHCl₃ (200 mL) and filtered through a 9.0 cm in diameter and 4 cm thick bed of silica gel, washed with CHCl₃ (2 x 100 mL) and filtrate was evaporated to dryness to afford crude 1-O-methyl-2-deoxy-4-thio-3,5-di-O-p-toluoyl-α,β-D-ribofuranose (formula 2) as a dark brown solid (24 g) which was dissolved in a acetolysis mixture containing acetic anhydride (200 mL), glacial acetic acid (200 mL), p-toluene sulfonic acid monohydrate (2.4 g) and conc. sulfuric acid (10 mL) and warmed to 40°C for 1 hour, then was decomposed by the addition of anhydrous sodium acetate. The resulting mixture was evaporated to dryness in vacuo at a temperature below 30°C. The residue was partitioned between 500 mL of water and 300 mL of CHCl₃. The aqueous phase was extracted with CHCl₃ (2 x 100 mL). The combined CHCl₃ layers were evaporated to dryness in vacuo, then several portions of methanol were added and removed in vacuo to eliminate the last traces of acetic anhydride. The residue was purified by a flash column containing 100 g of silica gel and eluted with 6:1 cyclohexane-ethylacetate and appropriate fractions were combined and evaporated to give a white solid, which was crystallized by 95% ethanol to give 1-O-acetyl-2-ceoxy-4-thio-3,5-di-O-p-toluoyl-α,β-D-ribofuranose, yield 18.26 g (70% from 1-O-methyl-2-deoxy-4-thio-α,β-D-ribofuranose) as a α,β mixture; MS z/e 429 (M + 1)⁺; ¹H NMR (CDCl₃, 300 MHz) δ 2.02 (s, 3, CH₃CO), 2.04 (s, 3, CH₃CO), 2.34 (s, 6, CH₃ of toluoyl), 2.36 (s, 6, CH₃ of toluoyl), 2.52-2.74 (m, 4, H-2), 3.92-4.06 (m, 2, H-4), 4.28-4.52 (m, 4, CH₂), 5.64-5.74 (m, 2, H-3), 6.12 (dd, 1, H-1 of β, J = 3 and 6 Hz ) , 6.2 (d, 1, H-1 of α, J = 5.5 Hz), 7.24-7.36 (m, 4, meta CH's of toluoyl), 7.8-7.92 (m, 4, ortho CH's of toluoyl). Anal. (C₂₃H₂₄O₆S), C, H, S.

### EXAMPLE 2

1-(2-Deoxy-4-thio-3,5-di-O-toluoyl-D-ribofuranosyl)uracil (formula 15). To a suspension of 1-O-acetyl-2-deoxy-4-thio-3,5-di-O-p-toluoyl-α,β-D--ribofuranose (formula 3) (428 mg, 1.0 mmol) and uracil(2,4-dioxopyrimidine) (112.1 mg, 1.0 mmol) in anhydrous 1,2-dichloroethane (30 mL) were added consecutively hexamethyldisilazane (HMDS, 161.5 mg, 1.0 mmol) and trimethylchlorosilane (TMSCl, 108.6 mg, 1.0 mmol) and the mixture was stirred at room temperature. After 0.5 hours, the resulting solution was cooled to -78°C and trimethylsilyl trifluoromethanesulfonate (266.7 mg, 1.2 mmol) was added to it and stirred at the same temperature for another 1 hour, after which time the reaction was essentially complete. The reaction mixture was warmed to room temperature and concentrated to a small volume (5 mL), diluted with methylene chloride (about 50 mL), then washed with water (15 mL) followed by saturated sodium bicarbonate and finally with water. The organic layer was dried over MgSO₄ and evaporated to dryness. The residue was purified by 50 g of silica gel with CHCl₃-MeOH 98:2 to afford a solid, which was crystallized from EtOH-dioxane to give pure 1-(2-deoxy-4-thio-3,5-di-O-toluoyl-D-ribofuranosyl)uracil (192 mg, 40%); mp 118-120°C; TLC 98:2 CHCl₃-MeOH; R_{f} 0.35; MS z/e 481 (M + 1)⁺; ¹H NMR (CDCl₃, 300 MHz) δ 2.40 (s, 6, CH₃ of toluoyl), 2.54-2.60 (m, 1, H-2'), 2.86-2.96 (m, 1, H-2'), 4.20-4.26 (m, 1, H-4'), 4.36-4.52 (m, 2, H-5'), 5.68-5.72 (m, 2, H-3', H-5), 6.44 (br d, 1, H-6, J = 6 Hz), 7.26 (d, 2, H's of toluoyl, J = 8 Hz), 7.30 (d, 2, H's of toluoyl, J = 8 Hz), 7.96 (d, 2, H's of toluoyl, J = 8 Hz), 8.14 (d, 2, H's of toluoyl, J = 8 Hz), 9.48 (s, 1, H-3); ¹³C NMR (Me₂SO-d₆, 300 MHz) δ 42.148 (C-2' ) , 56.46 (C-4'), 63. 55 (C-1' ) , 64.96 (C-5'), 78.21 (C-3' ) , 101.94 (C-5) , 126.03, 126.55, 129.23, 129.27, 129.783, 129.63 (toluoyl ring carbon), 141.74 (C-6), 144.14, 144..73 (toluoyl ring carbon), 150.71 (C-2), 163.05 (C-4), 165.36, 166.07 (carbonyl carbon of toluoyl).

### EXAMPLE 3

1-(2-Deoxy-4-thio-D-ribofuranosyl)uracil (formula 16). A solution of 1-(2-deoxy-4-thio-3,5-di-O-toluoyl-β-D-ribofuranosyl) uracil (formula 15) (175 mg, 0.36 mmol) in anhydrous MeOH (30 mL) was stirred at room temperature with a freshly prepared solution of sodium methoxide (39 mg, 0.72 mmol) in MeOH (7.5 mL). A TLC aliquot at 2.5 hours showed complete reaction. The solution was rendered neutral with Dowex 50W-X8 (H⁺) ion-exchange resin, the suspension filtered, and the resin was washed with MeOH. The filtrate was combined and evaporated to dryness, and methyl p-toluate was removed at 50°C/0.01 torr. Crystallization of the residue from absolute EtOH gave pure 1-(2-deoxy-4-thio-D-ribofuranosyl)uracil (75 mg, 85%), mp 190-192°C; TLC 9:1 CHCl₃-MeOH, R_{f} 0.30; MS z/e 245 (M + 1)⁺, UV λₘₐₓ PH 1 266 (9.24), pH 7 266 (9.52), pH 13 265 (8.72); ¹H NMR (Me₂SO-d₆, 300 MHz) δ 2.0-2.28 (m, 1, H-2'), 2.44-2.54 (m, 1, H-2'), 3.30-3.38 (m, 1, H-5' ) , 3.40-3.48 (m, 1, H-5' ) , 3.50-3.58 (m, 1, h-4'), 4.32 (br dd, 1, H-3', J = 2 and 8.Hz), 5.66 (d, 1, H-6, J = 8 Hz), 6.14 (dd, 1, H-1', J = 3 and 7.5 Hz), 8.26 (d, 1, H-5, J = 8 Hz); ¹³C NMR (Me₂SO-d₆, 300 MHz) δ 42.16 (C-2), 60.01 (C-4'), 60.95 (C-1'), 63.57 (C-5'), 74.14 (C-3' ) , 101.16 (C-5, J_{C,H} =175.24 Hz), 142.92 (C-6, J_{C,H} = 181.88 Hz), 150.69 (C-2), 162.97 (C-4). Anal. (C₉H₁₂N₄O₂S) C, H, N, S.

### EXAMPLE 4

1-(2-Deoxy-4-thio-3,5-di-O-toluoyl-β-D-ribofuranosyl)thymine (formula 17). To a suspension of 1-O-acetyl-2-deoxy-4-thio-3,5-di-O-p-toluoyl-α,β-D-ribofuranose (formula 3) (428 mg, 1.0 mmol), and thymine(5-methyl-2,4-dioxopyrimidine) (126 mg, 1.0 mmol) in anhydrous 1,2-dichloroethane (30 mL) were added consecutively hexamethyldisilazane (HMDS, 161.5 mg, 1.0 mmol), and trimethylchlorosilane (TMSC1, 108.6 mg, 1.0 mmol), and the mixture was stirred at room temperature after 0.5 hours. The resulting solution was cooled to -78°C and trimethylsilyl trifluoromethanesulfonate (266.7 mg, 1.2 mmol) was added to it and stirred at the same temperature for another 1.5 hours, after which time the reaction was essentially complete. The reaction mixture was warmed to room temperature and concentrated to a small volume (5 mL), diluted with methylene chloride (about 50 mL), then washed with water (15 mL) followed by saturated sodium bicarbonate and finally with water. The organic layer was dried over MgSO₄ and evaporated to dryness. The residue was purified by 50 g of silica gel with CHCl₃-MeOH 99:1 to afford a solid which was crystallized from EtOH-CHCl₃ to give pure 1-(2-deoxy-4-thio-3,5-di-O-toluoyl-β-D-ribofuranosyl)thymine (173 mg, 35%); mp 178-182°C; TLC 98:2 CHCl₃-MeOH, R_{f} 0.55; MS z/e 495 (M + 1)⁺; ¹H NMR (CDCl, 300 MHz) δ 1.78 (s, 3, C-5, CH₃'), 2.42 (s,3, CH₃ of toluoyl) 2.43 (s, 3, CH₃ of toluoyl), 2.36-2.44 (m, 1, H-2) , 3.98-4.04 (m, 1, H-4'), 4.12 (d, 2, H-5', J = 6 Hz), 5.76 (br t, 1, H-3'), 6.66 (dd, 1, H-1' , J = 6 and 9 Hz), 7.26 (d, 4, H's of toluoyl, J = 8 Hz), 7.56 (s, 1, H-6), 7.94 (d, 2, H's of toluoyl, J = 8 Hz), 7.96 (d, 2, H's of toluoyl, J = 8 Hz), 8.58 (s, 1, H-3); ¹³C NMR (CDCl₃, 300 MHz) δ 12.41 (C-5, CH₃), 21.69, 21.72 (CH₃ 's of toluoyl), 39.98 (C-2'), 53.13 (C-4', J_{C,H} = 148.1 Hz), 61.27 (C-1' , J_{C,H} = 163.3 Hz), 65.14 (C-5'), 77.13 (C-3'), 112.28 (C-5), 126.41, 126.58 (toluoyl ring carbon), 129.25, 129.35, 129.76, 129. 87 (toluoyl ring, carbon) , 135.4.6 (C-6, J_{C,H} = 176.46 Hz) , 144. 38, 144.47 (toluoyl ring carbon) , 150.45 (C-2) , 162.90 (C-4), 165.61, 166.17 (carbonyl carbon of toluoyl).

### EXAMPLE 5

1-(2'-Deoxy-4'-thio-β-D-ribofuranosyl)thymine (formula 18). A solution of 1-(2-deoxy-4-thio-3,5-di-O-toluoyl-β-D-ribofuranosyl) thymine (formula 17) (150 mg, 0.30 mmol) in anhydrous. MeOH (30 mL) was stirred at room temperature. with a freshly prepared solution of sodium methoxide (32.5 mg, 0.60 mmol) in MeOH (7.5 mL). A TLC aliquot at 3 hours showed complete reaction. The solution was rendered neutral with Dowex 50W-X8 (H⁺) ion-exchange resin, the suspension was filtered, and the resin was washed with MeOH. The filtrate was combined and evaporated to dryness, and methyl p-toluate was removed at 50°C/0.01 torr. Crystallization of the residue from absolute ETOH gave pure 1- (2'-deoxy-4'-thio-β-D-ribofuranosyl) thymine (61 mg, 78%), mp 213-215°C; TLC 9:1 CHCl₃-MeOH, R_{f} 0.40; MS z/e 258 (M + 1)⁺, UV λₘₐₓ pH 1 272 (10.3), pH 7 272 (10.2), pH 13 271 (10.3); ¹H NMR (Me₂ SO-d₆, 300 MHz) δ 1.80 (C-5, CH₃) , 2.10-2.24 (m, 2, H-2'), 3.24-3.32 (m, 1, H-4'), 3.50-3.66 (m, 2 H, H-5' ) , 4.38 (br s, 1, H-3') , 5.16 (br t, 1, 5'-OH), 5.24 (d, 1, 3'-OH, *J =* 4 Hz), 6.30 (dd, 1, H-1', J = 6.5 and 8 Hz) 7.32 (s, 1, H-6), 11.32 (br s, 1, H-3) ; ¹³C NMR (Me₂SO-d₆, 300 MHz) 6 12.16 (C-5, CH₃)' 40.97 (C-2' , J_{C,H} = 132.33 Hz) , 59.01 (C-4', J_{C,H} = 143.4 Hz), 59.93 (C-1',J_{C,H} = 167.74), 63.51 (C-5' ) , 73. 40 (C-3'), 109.82 (C-5) , 136.70 (C-6, J_{C,H} = 179 Hz) , 150.59 (C-4) , 163.37 (C-2). Anal. (C₁₀H₁₃N₂O₆S) C, H, N, S.

### EXAMPLE 6

### Antiviral activity of 2'-deoxy-4'-thioribonucleosides.

2'-Deoxy-4'-thioribonucleosides were tested for antiviral activity against viruses that replicate in mammalian cells growing in cell culture. The results of these tests against herpes simplex virus, Type 1 and Type 2, are summarized in Table 1. The Virus Rating (VR) is a standard weighted measurement of antiviral activity which takes into account the degree of inhibition of virus-induced cytopathogenic effects (CPE) and the degree of cytotoxicity produced by the test compound, determined by a modification of the method of Ehrlich et al, Ann. N.Y. Acad. Sci. 130, 5-16 (1965) .

The CPE-inhibition assays were designed to test seven 0.5 log₁₀ concentrations of each compound, beginning with 320 µg/mL, against HSV in triplicate 24-hour Vero cell monolayers in 96-well tissue culture plates. To each of the replicate cell cultures were dispensed 0.1 mL of the test compound solution (or suspension) and 0.1 mL of HSV suspension (diluted in medium to yield 32 CCID₅₀ units per 0.1 mL). Cell controls, untreated virus-infected controls and drug cytotoxicity controls were included in each assay. The plates were incubated at 37°C in a humidified atmosphere containing 2% CO₂ until 100% CPE were observed in the untreated virus control cultures. The cell monolayers were examined microscopically for drug cytotoxicity and for CPE. which was graded on a scale of 0-4 (0-100% CPE).

The VR was calculated as 0.1 of the sum of the numerical differences between the recorded CPE grade of each test well and that of the corresponding virus control in the culture plate. Numerical differences between the scores of test wells containing a drug concentration which is partially cytotoxic and their corresponding virus controls were halved.

In tests carried out by this method, a greater value of VR indicates greater antiviral activity. A compound with a VR of 1.0 or greater is considered to have significant antiviral activity with a high degree of reproducibility in confirmatory in vitro tests. A compound with a VR of 0.5-0.9 is considered to have possible or marginal activity; a compound with a VR of less than 0.5 is considered to be inactive.

The MIC₅₀ (minimum inhibitory concentration, 50%) is the concentration of a test compound required for 50% inhibition of virus-induced cytopathogenic effect calculated by using a regression analysis program for semilog curve fitting. MTC (minimum toxic concentration) is the minimum drug concentration (µg/ml) causing any cytotoxicity. TI is the therapeutic index, calculated by dividing the minimum cytotoxic drug concentration (MTC) by the minimum inhibitory concentration, 50% (MIC₅₀). The results were compared with two commercial antiviral agents, acyclovir and 9-β-D-arabinofuranosyladenine (Ara-A). The tests summarized in Table I show that definite antiviral activity against herpes simplex Type 1 is exhibited by two of the invention compounds, 1-(2-deoxy-4-thio-β-D-ribofuranosyl)thymine and 1-(2-deoxy-4-thio-α-D-ribofuranosyl)thymine.

**TABLE 1**

| Compound | Virus | VR | MIC₅₀ | MIC | TI |
|---|---|---|---|---|---|
| 1-(2-Deoxy-4-thio-α-D-ribofuranosyl)-thymine | HSV-1 HSV-2 | 1.4 0.3 | 100.1 - | >257.2 >257.2 | 2.3 - |
| 1-(2-Deoxy-4-thio-β-D-ribofuranosyl)-thymine | HSV-1 HSV-2 | 1. 3 0.1 | 0.8 - | 2.6 2.6 | 3.2 - |

| Controls | | | | | |
|---|---|---|---|---|---|
| Acyclovir | HSV-1 HSV-2 | 6.7 4.8 | 0.5 3.6 | >225.2 >225.2 | >441 63.2 |
| Ara-A | HSV-1 HSV-2 | 1.8 1.1 | 15.2 39.3 | 84.8 84.8 | 5.6 2.2 |
| ^{a/} HSV-1 (E377) ^{b/} HSV-2 (MS) | | | | | |

### EXAMPLE 7

2'-Deoxy-4'-thioribonucleosides were tested for antitumor activity against leukemia L1210 ("L1210") cells and human epidermoid carcinoma No. 2 ("H.Ep.-2") cells.

Table 2 sets forth the results of cytotoxicity tests. For L1210 cells the IC₅₀ is the concentration required to decrease cellular proliferation by 50% as compared to untreated controls. The cells were grown in suspension cultures and the number of cells present was determined at 24 and 48 hours. The values shown in Table 2 are 48 hour values.

For H.Ep.-2 cells, the IC₅₀ is the concentration required to reduce colony formation by 50% as compared to controls. One hundred cells in 10 mL of medium were placed in prescription bottles, and after 10 days incubation, the medium was decanted and the colonies were stained and counted.

In the cytotoxicity tests, the lower the IC₅₀ value, the greater the antitumor activity. An IC₅₀ value of less than 40 µg/mL indicates a compound of interest, and an IC₅₀ value of less than 1 indicates a compound that is extremely effective. As.shown in Table 2 below, all the compounds tested show an IC₅₀ value of less than 40 µg/mL with respect to either H.Ep.-2 or L1210 cells, or both. One compound, 1-(2-Deoxy-4-thio-β-D-ribofuranosyl) thymine shows an IC₅₀ value of much less than 1 µg/mL.

**TABLE 2**

| | IC₅₀ (µg/mL) | |
|---|---|---|
| Compound | H. Ep. -2 | L1210 |
| 1- (2-Deoxy-4-thio-β-D-ribofuranosyl)thymine | 0.075 | 0.025 |
| 1- (2-Deoxy-4-thio-α-D-ribofuranosyl)thymine | 2.7 | 20 |

Although the invention has been described in considerable detail with specific reference to certain advantageous embodiments thereof, variations and modifications can be made without departing from the scope of the invention as defined in the appended claims.

## Claims

1. A β-2'-deoxy-thioribonucleoside represented by the formula: 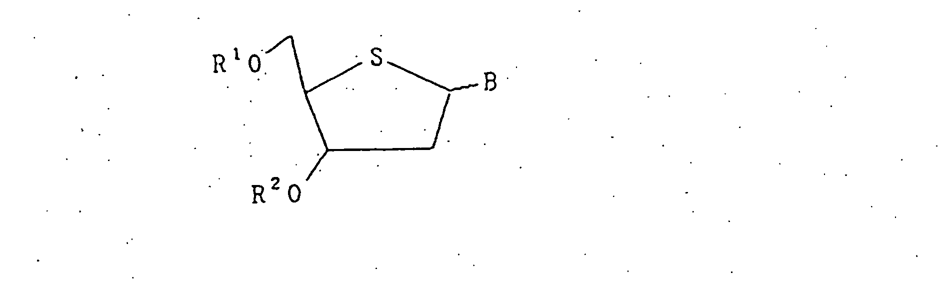 which B is a pyrimidine, 5-azapyrimidine, 6-azapyrimidine or 3-deazapyrimidine base, and R¹ and R² may be the same or different and are hydrogens or acyl protecting groups wherein the substituent on C-5 of B is either hydrogen or a methyl group,
provided that when B is a pyrimidine base, it is not uracil having a methyl group attached to the C-5 heterocyclic carbon.

2. A β-2'-deoxy-thioribonucleoside represented by the formula: 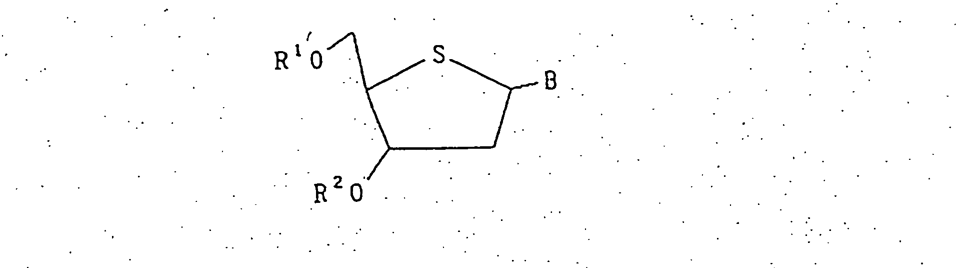 which B is of the following nitrogenous heterocyclic bases: 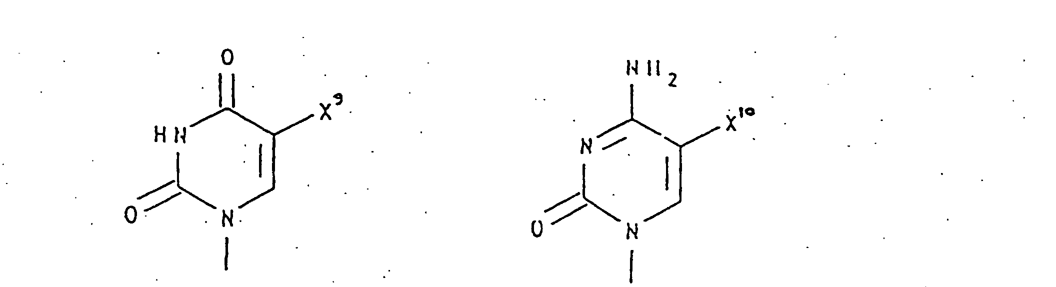 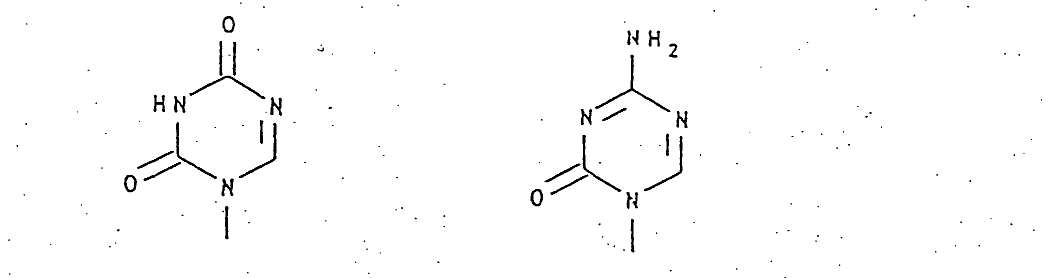 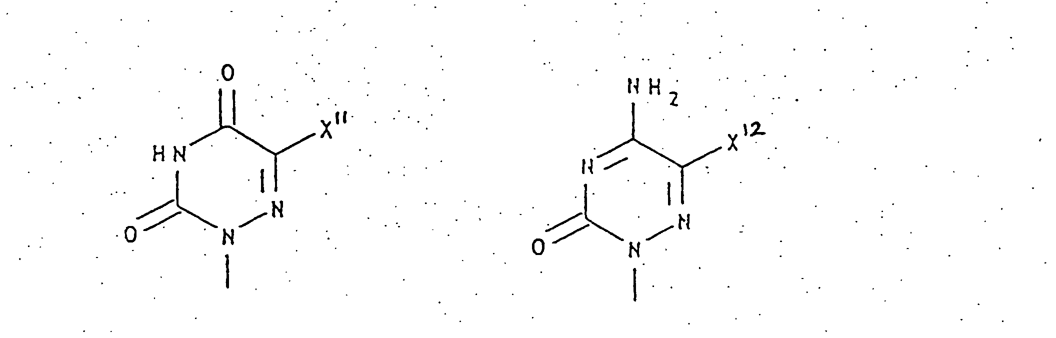 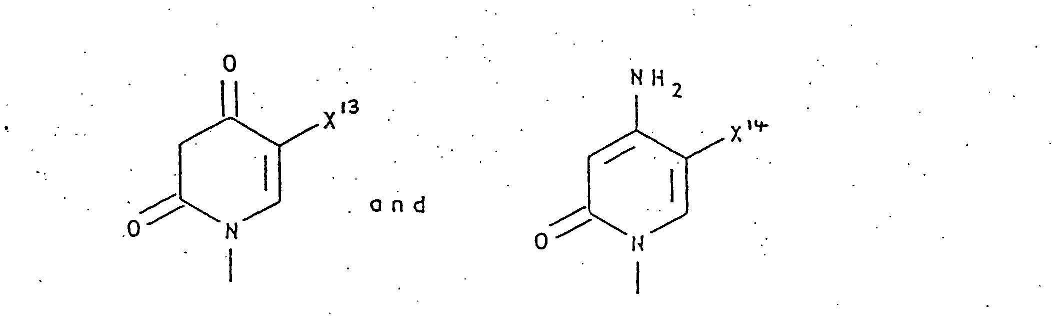 where X⁹, X¹⁰, X¹¹, X¹² , X¹³, X¹⁴, are each hydrogen ,or methyl, and R¹ and R² can be the same or different and are hydrogen or acyl protecting groups, provided that it is not uracil having a methyl group attached to the C⁵ heterocyclic carbon.

3. The β-2'-deoxy-thioribonucleoside of claim 1, wherein B is a pyrimidine base.

4. A β-2'-deoxy-4'-thioribonucleoside represented by the formula: 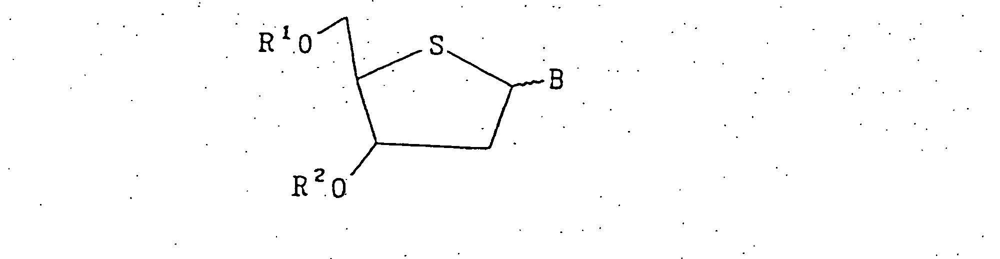 which B is one of the following pyrimidine bases: 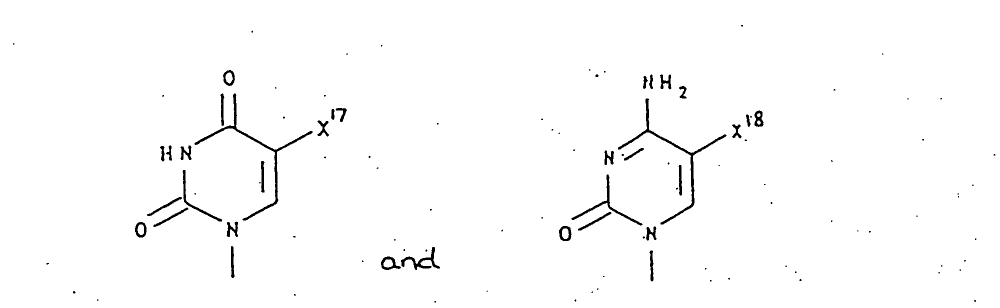 where X¹⁷ and X¹⁸ are hydrocarbon, or methyl , and R¹ and R² may be the same or different and are hydrogens or acyl protecting groups provided that it is not uracil having a methyl attached to the C⁵ heterocyclic carbon.

5. A ribonucleoside as claimed in any preceding claim which each of R¹ and R² is hydrogen.

6. 1-(2-Deoxy-4-thip-β-D-ribofuranosyl) uracil.

7. 1-(2-deoxy-4-thio-3,5-di-O-toluoyl-β-D-ribofuranosyl) uracil.

8. 1- (2-deoxy-4-thio-3,5-di-O-toluoyl-β-D-ribofuranosyl) thymine.

9. A pharmaceutical preparation comprising a therapeutically effective amount of a compound as claimed in any of claims 1 to 6.

10. The use of a compound as claimed in any of claims 1 to 6 for the manufacture of a medicament for the treatment of a host animal having a viral infection.

11. The use claimed in Claim 10 in which the virus infection is a herpes simplex virus infection.

12. The use of a compound as claimed in any of claims 1 to 6 for the manufacture of a medicant for the treatment of an animal afflicted with cancer.

## Patentansprüche

1. β-2'-Desoxy-thioribonucleosid mit der Formel 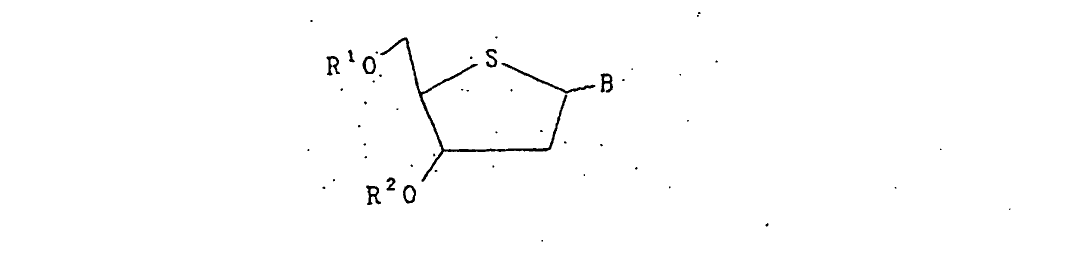 worin B eine Pyrimidin-, 5-Azapyrimidin-, 6-Azapyrimidin- oder 3 Desazapyrimidinbase ist und R¹ und R² gleich oder verschieden sein können und Wasserstoffatome oder Acylschutzgruppen sind, wobei der Substituent an C-5 von B entweder Wasserstoff oder eine Methylgruppe ist, mit dem Vorbehalt, dass dann, wenn B eine Pyrimidinbase ist, diese Base nicht Uracil ist, bei dem eine Methylgruppe an das heterocyclische C-5 Kohlenstoffatom gebunden ist.

2. β-2'-Desoxy-thioribonucleosid der Formel 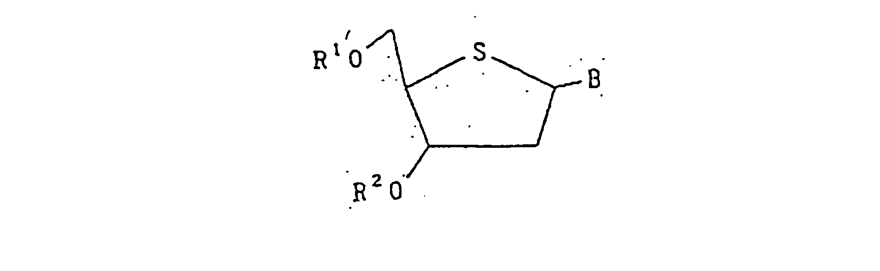 wobei B eine der folgenden stickstoffhaltigen heterozyklischen Basen ist: 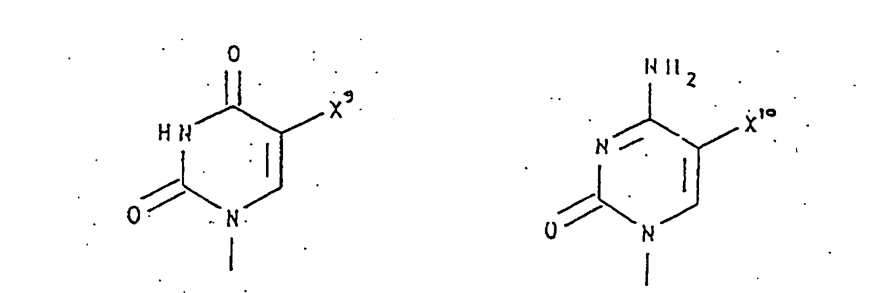 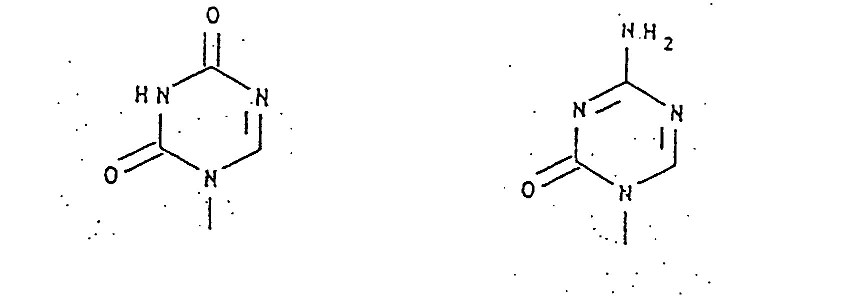 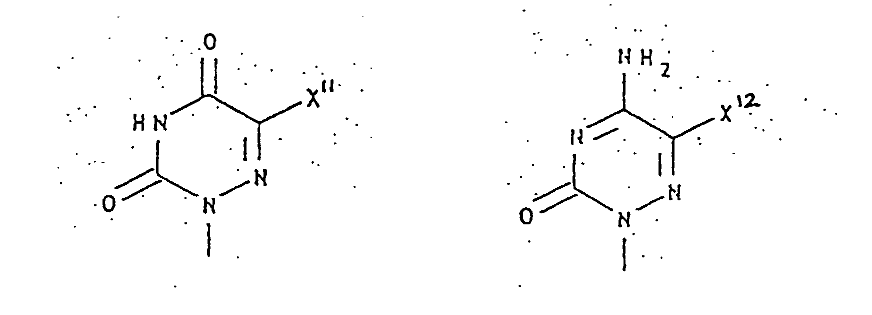 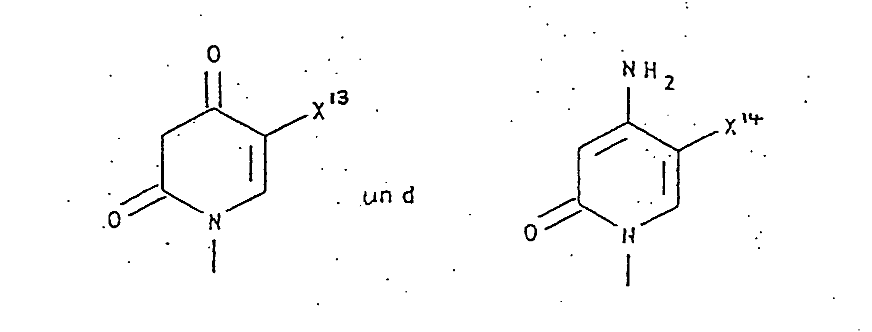 worin X⁹, X¹⁰, X¹¹, X¹², X¹³, X¹⁴ jeweils Wasserstoffatome oder Methylreste sind und R¹ und R² gleich oder verschieden sein können und Wasserstoff oder Acylschutzgruppen sind, mit dem Vorbehalt, dass B nicht Uracil ist, bei dem eine Methylgruppe an den heterocyclischen C-5-Kohlenstoff gebunden ist.

3. β-2'-Desoxy-thioribonucleosid nach Anspruch 1, wobei B eine Pyrimidinbase ist.

4. β-2'-Desoxy-4'-thioribonucleosid der Formel 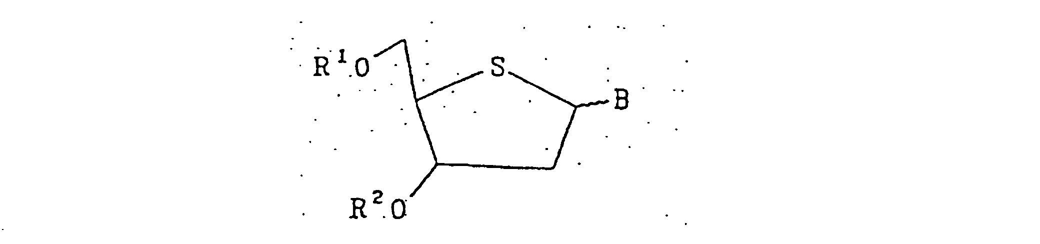 wobei B eine der folgenden Pyrimidinbasen ist: 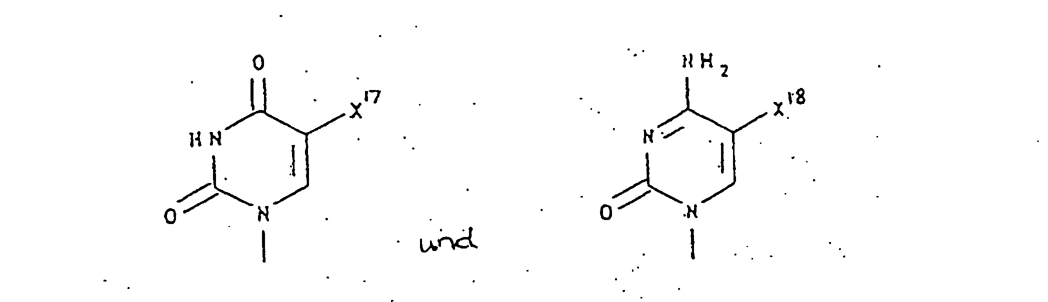 worin X¹⁷ und X¹⁸ Kohlenwasserstoffe oder Methylreste sind und R¹ und R² gleich oder verschieden sein können und Wasserstoffatome oder Acylschutzgruppen sind, mit dem Vorbehalt, dass B nicht Uracil ist, bei dem an das heterocyclische C-5-Kohlenstoffatom eine Methylgruppe gebunden ist.

5. Ribonucleosid, wie in einem der vorhergehenden Ansprüche beansprucht, bei dem jeder der Reste R¹ und R² Wasserstoff ist.

6. 1-(2-Desoxy-4-thio-*β*-D-ribofuranosyl) uracil.

7. 1-(2-Desoxy-4-thio-3,5-di-O-toluoyl-β-D-ribofuranosyl) uracil.

8. 1-(2-Desoxy-4-thio-3,5-di-O-toluoyl-β-D-ribofuranosyl) thymin.

9. Pharmazeutisches Präparat enthaltend eine therapeutisch wirksame Menge einer Verbindung nach einem der Ansprüche 1 bis 6.

10. Verwendung einer Verbindung nach einem der Ansprüche 1 bis 6 zur Herstellung eines Arzneimittels zur Behandlung eines Wirtstieres mit einer viralen Infektion.

11. Verwendung nach Anspruch 10, wobei die Virusinfektion eine Infektion mit Herpes Simplex-Virus ist.

12. Verwendung einer Verbindung, wie in einem der Ansprüche 1 bis 6 beansprucht, zur Herstellung eines Arzneimittels zur Behandlung eines an Krebs leidenden Tieres.

## Revendications

1. β-2'-désoxy-thioribonucléoside représenté par la formule : 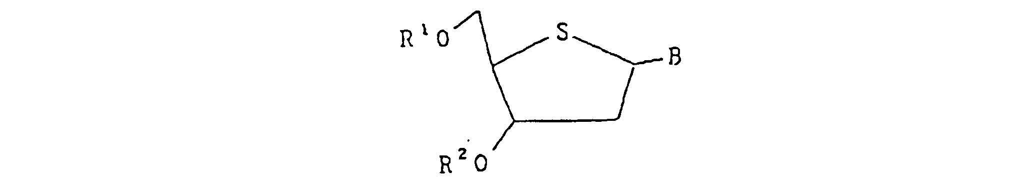 où B est une base pyrimidine, 5-azapyrimidine, 6-azapyrimidine ou 3-déazapyrimidine, et R¹ et R² peuvent être identiques ou différents et sont des hydrogènes ou des groupements acyle protecteurs,
où le substitut sur C-5 de B est soit de l'hydrogène, soit un groupement méthyle,
à condition que, lorsque B est une base pyrimidine, ce ne soit pas de l'uracile comportant un groupement méthyle fixé sur le carbone hétérocyclique C-5.

2. β-2'-désoxy-thioribonucléoside représenté par la formule : 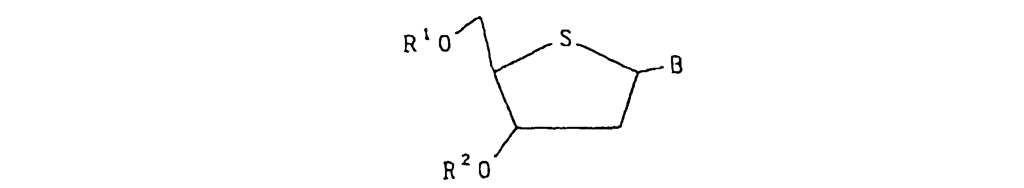 où B est l'une des bases hétérocycliques azotées suivantes : 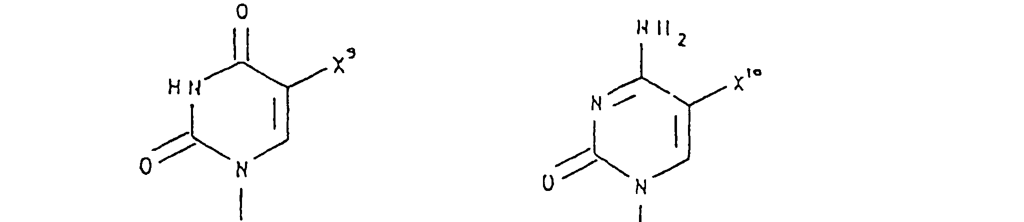 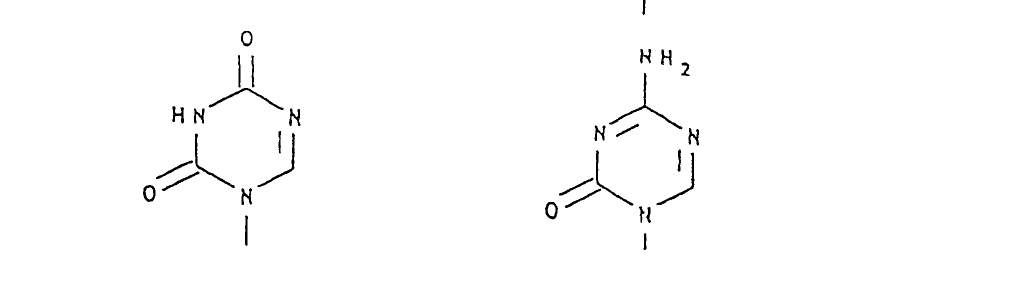 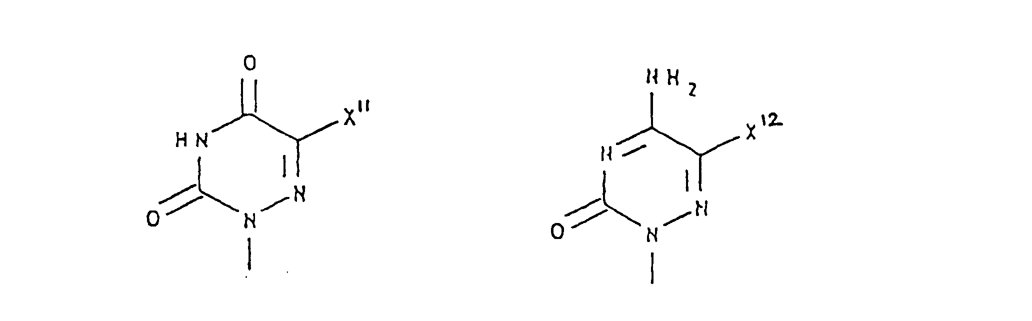 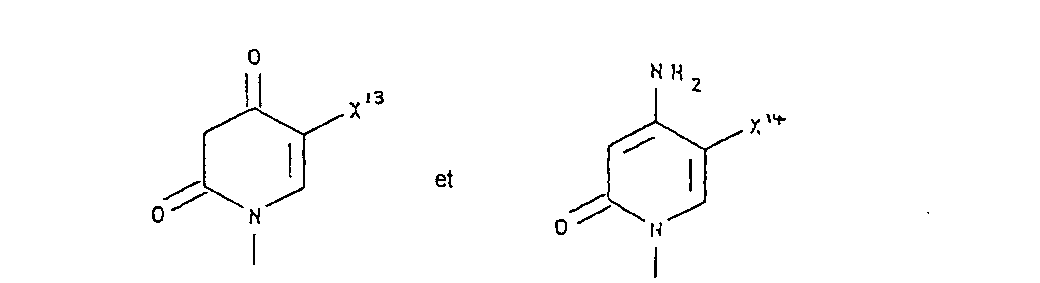 où X⁹, X¹⁰, X¹¹, X¹², X¹³, X¹⁴ sont chacun de l'hydrogène ou un groupement méthyle, et R¹ et R² peuvent être identiques ou différents et sont de l'hydrogène ou des groupements acyle protecteurs, à condition que ce ne soit pas de l'uracile comportant un groupement méthyle fixé sur le carbone hétérocyclique C⁵.

3. β-2'-désoxy-thioribonucléoside selon la revendication 1, où B est une base pyrimidine.

4. β-2'-désoxy-4'-thioribonucléoside représenté par la formule 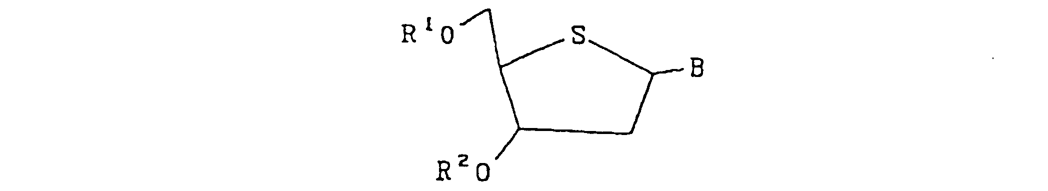 où B est l'une des bases pyrimidine suivantes : 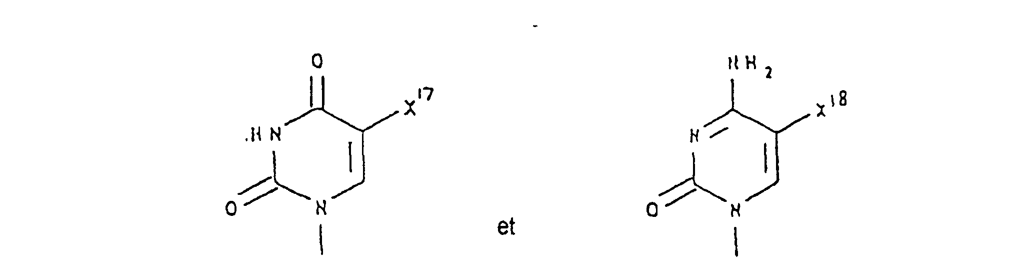 où X¹⁷ et X¹⁸ sont un hydrocarbure ou un groupement méthyle, et R¹ et R² peuvent être identiques ou différents et sont des hydrogènes ou des groupements acyle protecteurs, à condition que ce ne soit de l'uracile comportant un groupement méthyle fixé sur le carbone hétérocyclique C⁵.

5. Ribonucléoside selon l'une quelconque des revendications précédentes, où chacun de R¹ et de R² est de l'hydrogène.

6. 1-(2-désoxy-4-thio-β-D-ribofuranosyl) uracile.

7. 1-(2-désoxy-4-thio-3,5-di-O-toluoyl-β-D-ribofuranosyl) uracile.

8. 1-(2-désoxy-4-thio-3,5-di-O-toluoyl-β-D-ribofuranosyl) thymine.

9. Préparation pharmaceutique comprenant une quantité thérapeutiquement efficace d'un composé selon l'une quelconque des revendications 1 à 6.

10. Utilisation d'un composé selon l'une quelconque des revendications 1 à 6 pour la fabrication d'un médicament destiné au traitement d'un animal hôte présentant une infection virale.

11. Utilisation selon la revendication 10, dans laquelle l'infection virale est une infection par le virus de l'herpès simplex.

12. Utilisation d'un composé selon l'une quelconque des revendications 1 à 6 pour la fabrication d'un médicament destiné au traitement d'un animal atteint d'un cancer.
